# EUROPEAN PATENT SPECIFICATION

(11) **EP 3 154 639 B1**
(45) Date of publication and mention of the grant of the patent: **02.09.2020**
(21) Application number: 15805995.6
(22) Date of filing: 12.06.2015
(51) Int. Cl.: A61K 38/17, A61K 38/37, A61K 39/395, C07K 16/28, C07K 16/30, A61P 7/00

(54) **USES OF HUMANIZED COBRA VENOM FACTOR FOR REDUCING OR PREVENTING IMMUNOGENICITY**
VERWENDUNGEN VON HUMANISIERTEM KOBRAGIFTFAKTOR ZUR VERRINGERUNG ODER VERHINDERUNG VON IMMUNOGENITÄT
UTILISATIONS DU FACTEUR DE VENIN DE COBRA HUMANISÉ POUR RÉDUIRE OU PRÉVENIR L'IMMUNOGÉNICITÉ

(30) Priority: 12.06.2014 US 201462011236 P
(43) Date of publication of application: 19.04.2017
(73) Proprietor: University Of Hawaii, Honolulu, HI 96822 (US)
(72) Inventor: VOGEL, Carl-Wilhelm, Honolulu, HI 96813 (US); FINNEGAN, Paul, Del Mar, CA 92014 (US); RAYES, Julie, F-75006 Paris (FR); LACROIX-DESMAZES, Sébastien, F-75006 Paris (FR)
(74) Representative: Comoglio, Elena
(86) International application number: PCT/US2015/035693
(87) International publication number: WO 2015/192101

(56) References cited:
- WO-A1-2012/058480
- WO-A1-2013/106789
- US-A1- 2010 179 092
- US-B2- 8 632 780
- CARL-WILHELM VOGEL ET AL: "Cobra venom factor: Structure, function, and humanization for therapeutic complement depletion", TOXICON, vol. 56, no. 7, 1 December 2010 (2010-12-01), pages 1198-1222, XP055046258, ISSN: 0041-0101, DOI: 10.1016/j.toxicon.2010.04.007
- ALEXANDER RÖTH ET AL: "Chronic treatment of paroxysmal nocturnal hemoglobinuria patients with eculizumab: safety, efficacy, and unexpected laboratory phenomena", INTERNATIONAL JOURNAL OF HEMATOLOGY, SPRINGER JAPAN, JAPAN, vol. 93, no. 6, 25 May 2011 (2011-05-25), pages 704-714, XP019913226, ISSN: 1865-3774, DOI: 10.1007/S12185-011-0867-Y
- HILLMEN P ET AL: "EFFECT OF ECULIZUMAB ON HEMOLYSIS AND TRANSFUSION REQUIREMENTS IN PATIENTS WITH PAROXYSMAL NOCTRURNAL HEMAGLOBINURIA", NEW ENGLAND JOURNAL OF MEDICINE, THE -, MASSACHUSETTS MEDICAL SOCIETY, US, vol. 350, no. 6, 5 February 2004 (2004-02-05), pages 552-559, XP009057948, ISSN: 1533-4406, DOI: 10.1056/NEJMOA031688
- CARL-WILHELM VOGEL ET AL: "Humanized cobra venom factor: Structure, activity, and therapeutic efficacy in preclinical disease models", MOLECULAR IMMUNOLOGY., vol. 61, no. 2, 1 October 2014 (2014-10-01), pages 191-203, XP055423384, GB ISSN: 0161-5890, DOI: 10.1016/j.molimm.2014.06.035
- WANG, SY ET AL.: 'Depletion Of The C3 Component Of Complement Enhances The Ability Of Rituximab-Coated Target Cells To Activate Human NK Cells And Improves The Efficacy Of Monoclonal Antibody Therapy In An In Vivo Model.' BLOOD vol. 114, no. 26, 17 December 2009, ISSN 0006-4971 pages 5322 - 5330, XP055046784
- GORSUCH, BW ET AL.: 'Humanized Cobra Venom Factor Decreases Myocardial Ischemia Reperfusion Injury.' MOL IMMUNOL. vol. 47, no. 2-3, December 2009, ISSN 0161-5890 pages 50 - 510, XP026886972

## Description

### CROSS-REFERENCE TO RELATED APPLICATIONS

This application claims the benefit of U.S. Provisional Patent Application No. 62/011,236, filed June 12, 2014.

### FIELD OF THE INVENTION

This disclosure relates to compositions and methods for complement depletion and for reducing or preventing immunogenicity and other unwanted immunological reactions.

### BACKGROUND

The complement system is an integral component of both innate and adaptive immunity. However, complement is also a pathogenetic factor in many diseases. The development of agents for therapeutic complement inhibition is the topic of intense investigations by many investigators.

Biologics therapy are composed of protein or protein-containing agents, such as recombinant protein, fusion protein, monoclonal antibodies from variety of sources, medicines derived from human, animal, plants, viruses, bacteria, microorganisms and other living matter.

Biologics agents also comprise new methods of treatments such as gene therapy with viral vehicles, engineered or minimally manipulated cells such as single-lineage cell transplants, for example mesenchymal stromal (stem) cells, pancreatic islet cells and hepatocytes and other therapies assembled from amino acids, both natural and unnatural, including cellular or protein based medical devices.

While many biologic therapies are safe and effective for treating variety of disease or deficiencies in humans and animals, they can induce unwanted immunogenicity, thus rendering ineffective the biologic therapy, or cause adverse events such as anaphylaxis acutely and autoimmune disease chronically.

In many therapeutic situations, biologics agents may induce unwanted immunogenicity such as neutralizing antibodies or anaphylaxis, which can cause substantial clinical challenges for the physician and the patient. Neutralizing antibodies can render a potentially effective biologic therapy ineffective. Unwanted immunological reaction can destroy biological therapy. Anaphylaxis can be life threatening. Therefore, there remains a need for more effective and/or safer methods for prevention of immunogenicity related to biologics therapy.

### SUMMARY

There are disclosed methods of complement depletion and for reduction or prevention of immunogenicity, especially on a repeated basis. This is especially desired during biologics therapy because such therapy can generate an immune response that interferes with the efficacy of the therapy.

Hence, an embodiment of the invention is directed to humanized cobra venom factor (hCVF) for use according to appended claim 1 or 8. The administration of the agent and hCVF can be a second or a subsequent administration to the subject, with the immune response being reduced or prevented by the second or subsequent administration. Preferably, the hCVF is administered prior to administration of the biological therapeutic agent.

The depletion of complement levels in the subject may be affected for different amounts of time. Thus, for example, complement levels may return to pre-hCVF administration levels within about 12 hours to 24 hours, within about 5 days to 7 days, or return in greater than 7 days. Furthermore, the route of hCVF administration may be selected from one or more of administration into an organ, into a cavity, into a tissue, intravenous, intraperitoneal, intraarterial, and subcutaneous administration.

According to the invention, hCVF is used for treating a blood clotting condition or disease associated with unwanted complement activation in a subject. Treatment includes administering to the subject a humanized cobra venom factor (hCVF) in an amount sufficient to deplete complement. Moreover, treatment may include administration of the hCVF for a second or subsequent time (i.e., on a repeated basis) to the subject, with complement being depleted by each administration. Such conditions or diseases include, for example, a blood clotting disorder, such as paroxysmal nocturnal hemoglobinuria, hemophilia A and atypical hemolytic ureic syndrome.

The use of hCVF in the reduction or prevention of immunogenicity in a subject, including a second or subsequent use in the subject with the effect of reducing or preventing immunogenicity during each use, also is contemplated, as well as the use of hCVF in the manufacture of a medicament for depleting complement, with the medicament depleting complement during each use in a subject in need thereof.

These and other features, aspects, and advantages of the present invention will become better understood upon consideration of the following detailed description, drawings and appended claims.

### BRIEF DESCRIPTION OF THE FIGURES

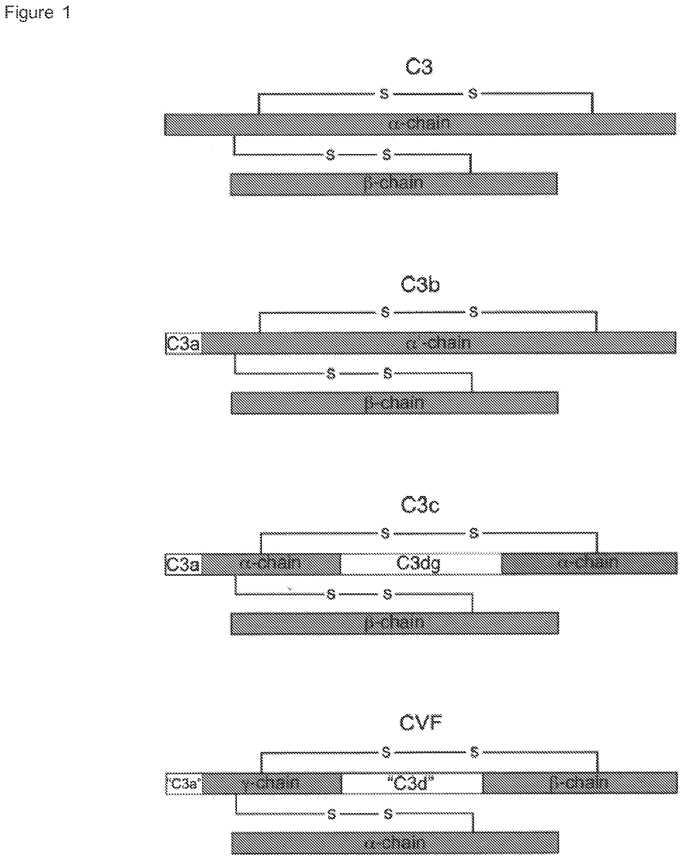

Figure 1. Schematic representation of the chain structures of C3, C3b, C3c, and cobra venom factor (CVF). The CVF γ- and β-chains are larger than the corresponding α'-chain fragments of C3c.

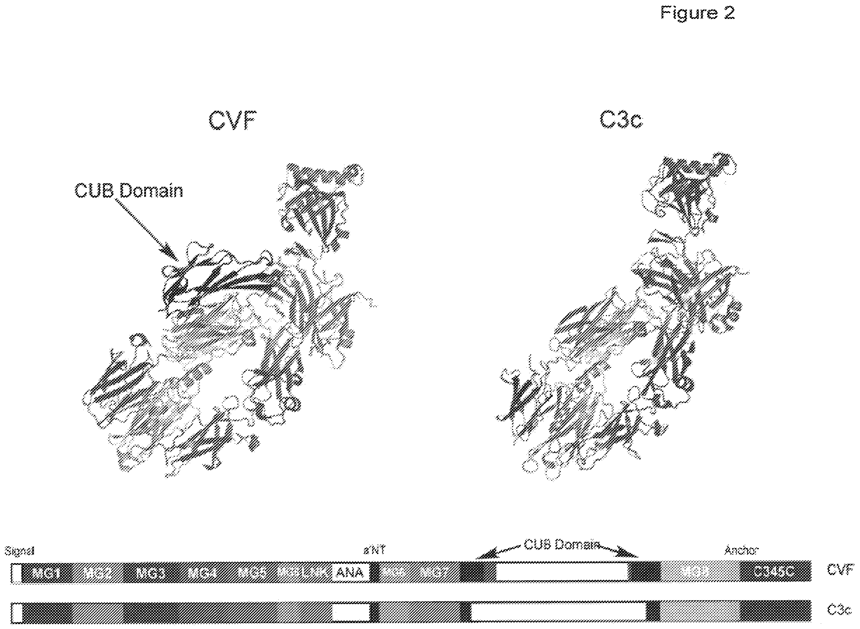

Figure 2. Crystal structures of CVF and C3c. The upper panel shows the three- dimensional domain structures of CVF (2.2 Å) and C3c (2.4 Å). The lower panel shows the schematic linear domain structures. Please note the absence of the CUB domain in C3c compared to CVF.

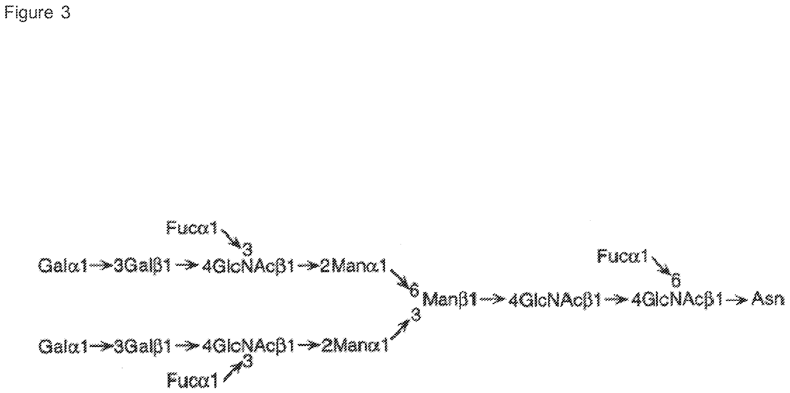

Figure 3. Structure of the major oligosaccharide chain of CVF displaying α-galactosylated Le^{x} structures.

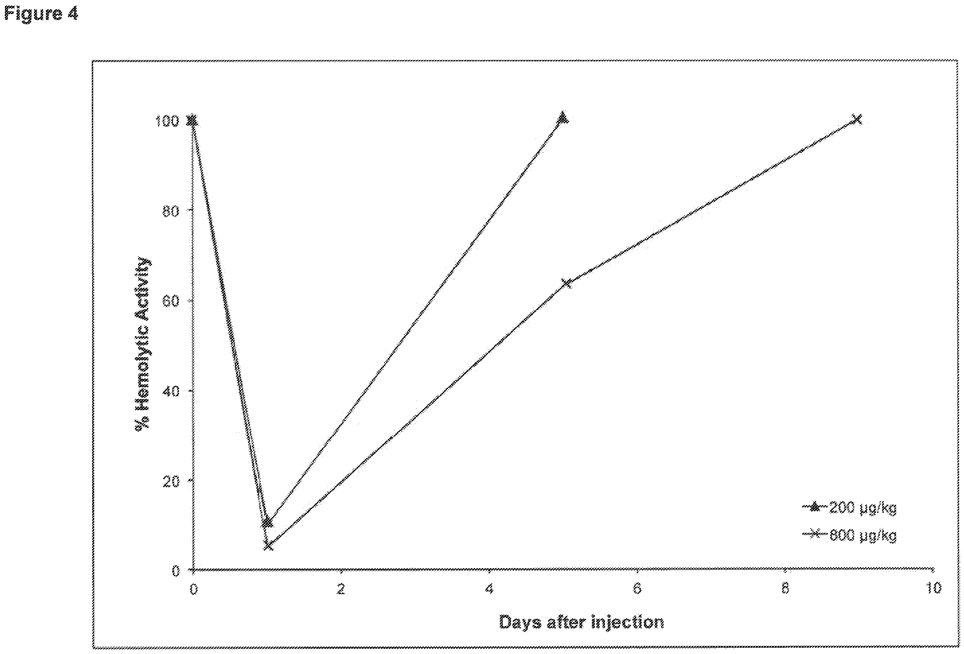

Figure 4. *In vivo* complement depletion by CVF. Mice were injected i.p. with a single dose of CVF as shown. At the time intervals indicated, the hemolytic complement activity in serum was measured.

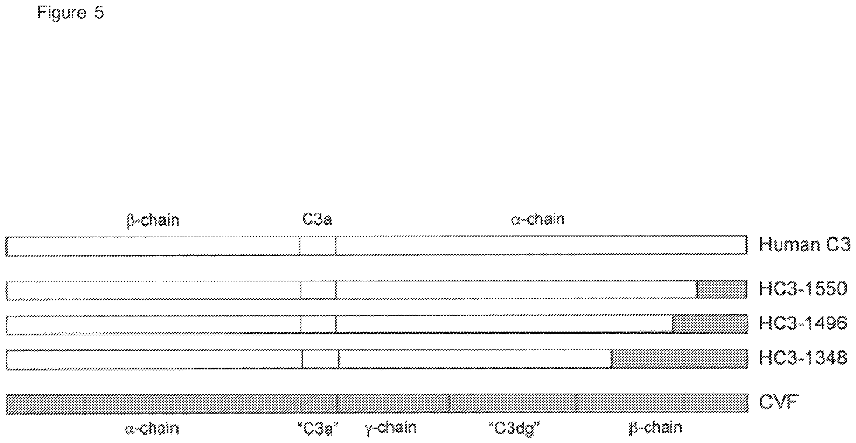

Figure 5. Schematic representation of the chain structures of humanized CVF (hCVF) proteins. The N-terminus is to the left. Chain homologies with human C3 and CVF are indicated.

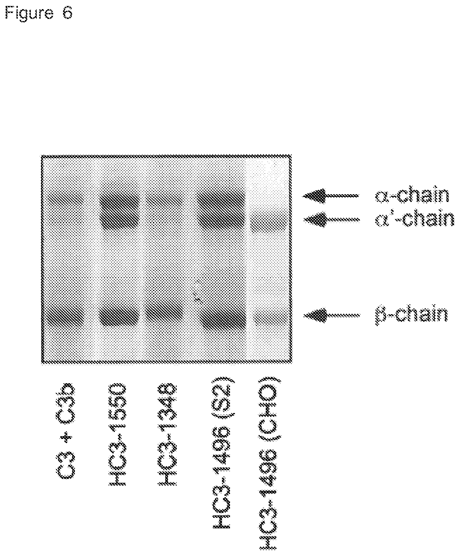

Figure 6. Chain structures of recombinantly produced hCVF proteins. Shown are Coomassie-stained SDS polyacrylamide gels under reducing conditions of hCVF proteins HC3-1550, HC3-1348, and HC3-1496 produced in S2 cells as well as HC3-1496 produced in CHO cells. A mixture of C3 and C3b is shown as control.

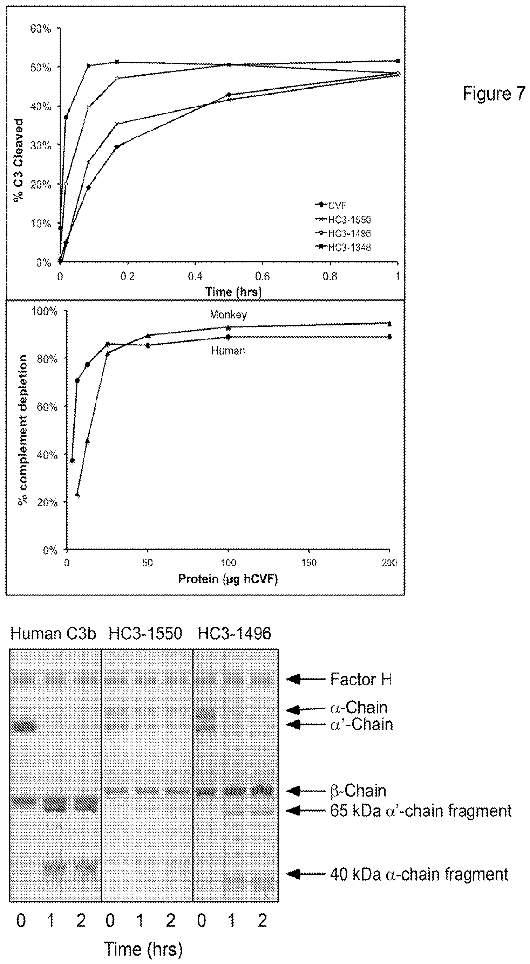

Figure 7. Functional properties of hCVF. Upper panel: C3-cleaving activity. Shown is a time course of cleavage of purified human C3 by convertases preformed with hCVF proteins or CVF is indicated. Center panel: *in vitro* complement depletion by hCVF protein HC3-1496. Shown are dose response curves in human and monkey serum. Bottom panel: partial resistance to degradation by Factors H and I of hCVF proteins. Shown are Coomassie-stained SDS polyacrylamide gels of aliquots taken at time intervals as indicated of reaction mixtures containing purified human Factors H and I and hCVF proteins. Human C3b served ,as control. Proteolytic degradation of C3b and hCVF proteins is demonstration by the disappearance of the α- and α'-chains, and the appearance of corresponding α-chain fragments. Note that significant amounts of both α-chain and α'-chain of hCVF proteins are still present after two hours of incubation, in contrast to the rapid disappearance of the C3 α'-chain of C3b.

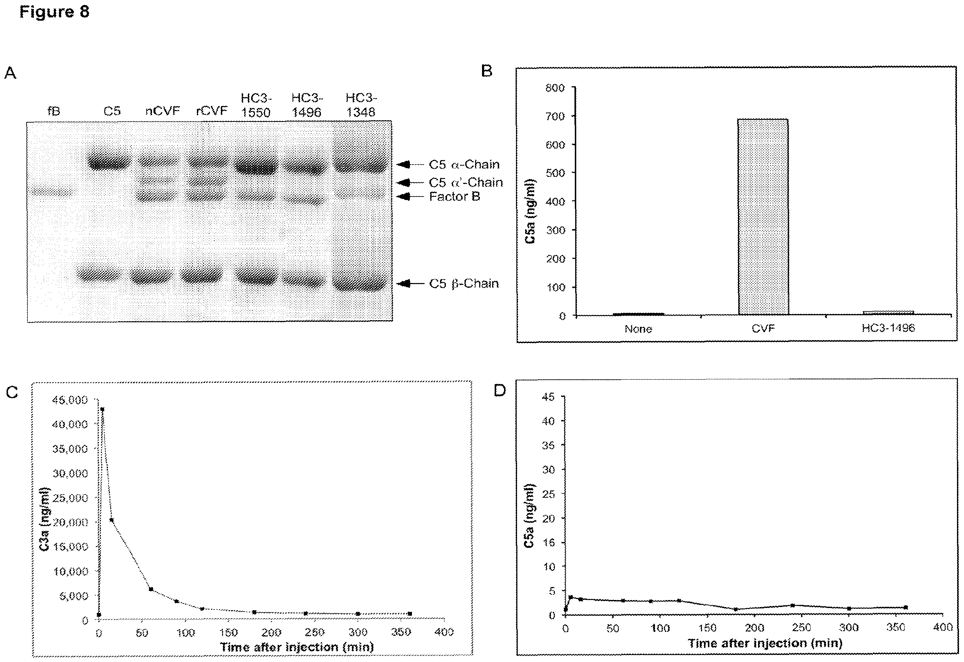

Figure 8. Lack of C5-cleaving activity of hCVF proteins. Panel A: shown are Coomassie-stained SDS polyacrylamide gels under reducing conditions of incubation mixtures containing purified human C5, EDTA, and convertases preformed with several hCVF proteins. Natural CVF and rCVF are included as positive controls, demonstrating the disappearance of the C5 a-chain and corresponding appearance of the C5 α'-chain. The two left lanes show purified human Factor 8 and C5 as a controls. Panel B: shown is the generation of the C5a anaphylatoxin in cynomolgus monkey serum *in vitro* by complement depletion with natural CVF or hCVF protein HC3-1496 as measured by ELISA. Panels C and D: shown is the generation of the C3a (Panel C) and C5a anaphylatoxins (Panel D) in cynomolgus monkey after injection of hCVF protein HC3-1496 at 1,000 µg/kg into the pulmonary artery. Anaphylatoxins were measured by ELISA at time intervals as indicated. C5a levels were at the limit of detectability of the assay; please note the 1,000-fold difference in the scale of the Y-axis in the two panels.

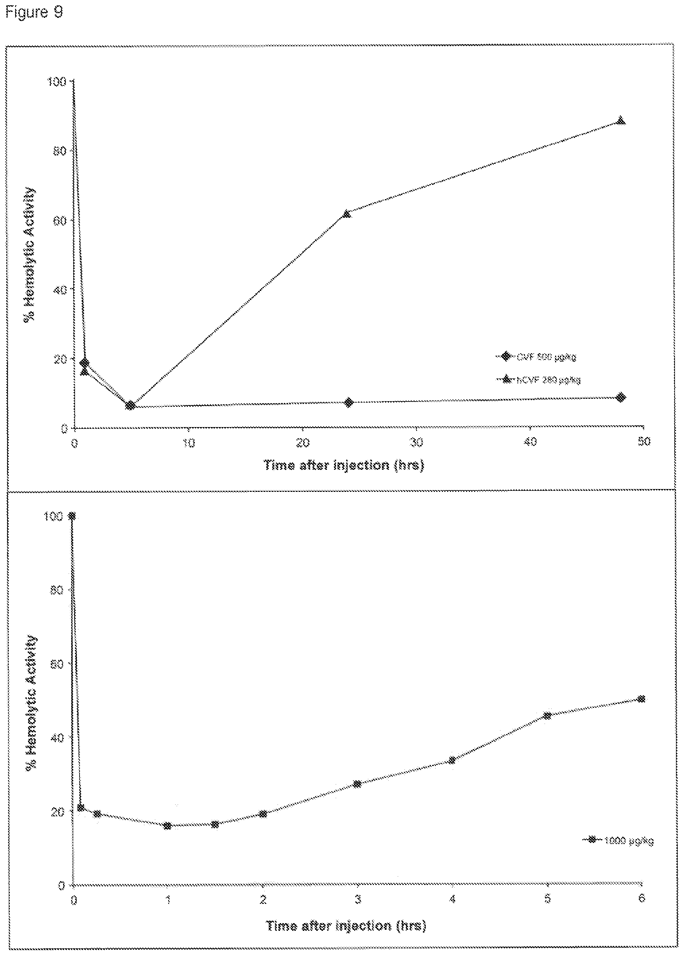

Figure 9. *In vivo* complement depletion by hCVF. Shown is a time course of complement depletion in rat after i.p. injection of HC3-1496 at 280 µg/kg (upper panel) and in cynomolgus monkey after intra-arterial injection of HC3-1496 at 1,000 µg/kg (lower panel). Please note the rapid depletion of complement within minutes of injection. Please also note the longer period of complement depletion by natural CVF.

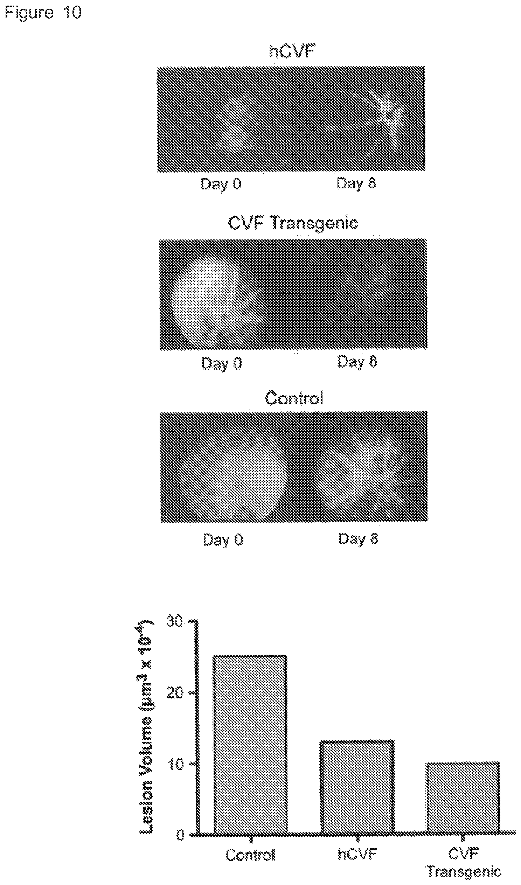

Figure 10. Effect of complement depletion with hCVF in a murine models of age- related macular degeneration (AMD) induced by laser photocoagulation. The upper panel shows funduscopic images after i.v. injection of FITC-dextran on days 0 and 8. The lower panel shows the lesion volume at day 28 as determined by histopathological examination. Please note the significantly smaller lesions in hCVF-treated mice and CVF transgenic mice compared to PBS-treated controls.

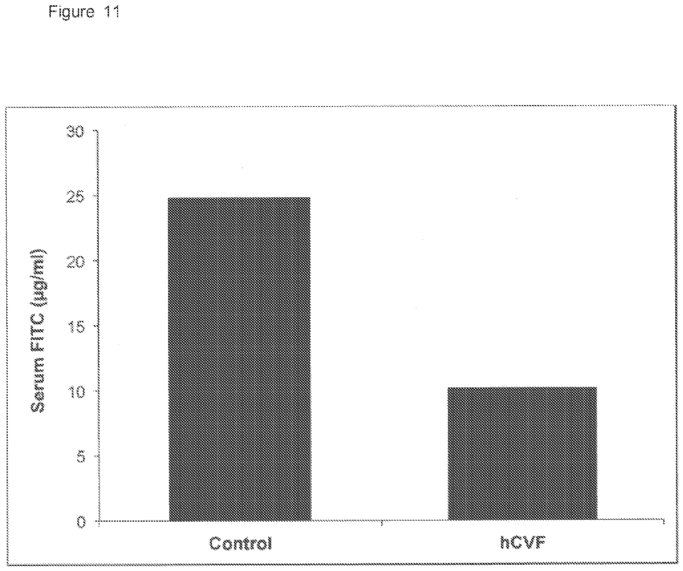

Figure 11. Effect of complement depletion with hCVF in a murine model of gastrointestinal ischemia reperfusion injury. Shown is the concentration of FITC-conjugated dextran in the serum after ischemia in hCVF-treated and control animals. Please note the significantly decreased concentration of FITC-dextran in complement-depleted mice, indicating a significantly reduced extent of the intestinal reperfusion injury.

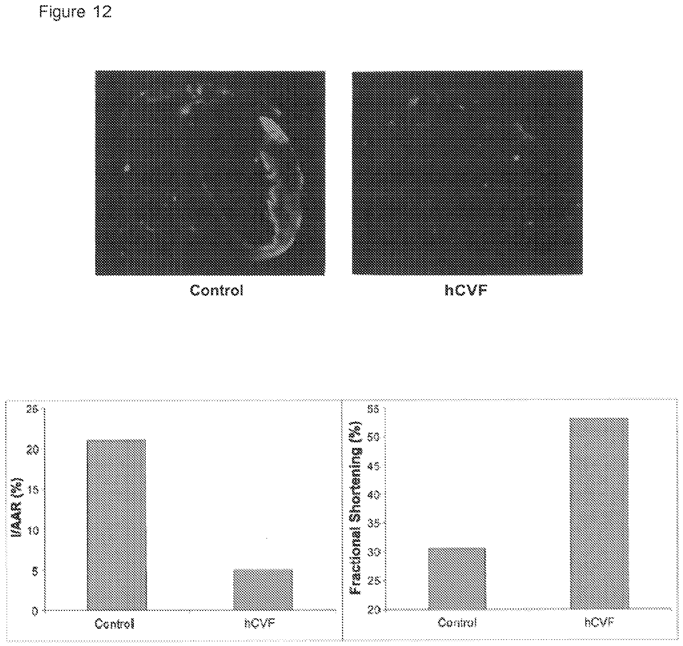

Figure 12. Effect of complement depletion with hCVF in a murine model of myocardial ischemia reperfusion injury. The upper panel shows microscopic images after immunohistochemical staining for C3b deposition. The left lower panel shows the size of the infarcted area as a percentage of the area at risk. The right lower right panel shows the fractural shortening as a measure of left ventricular function as determined by echocardiography.

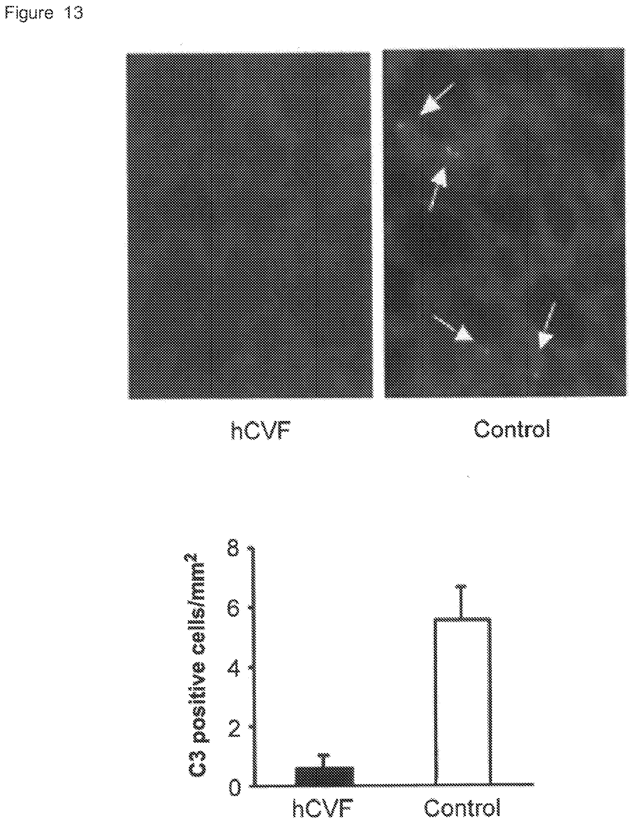

Figure 13. Effect of complement depletion with hCVF in a murine model of ventilator-induced lung injury (VILI). The upper panels shows microscopic images after immunohistochemical staining for C3b deposition. White arrows indicate C3b-positive cells. The lower panel shows a quantitative analysis of C3b-positive cells.

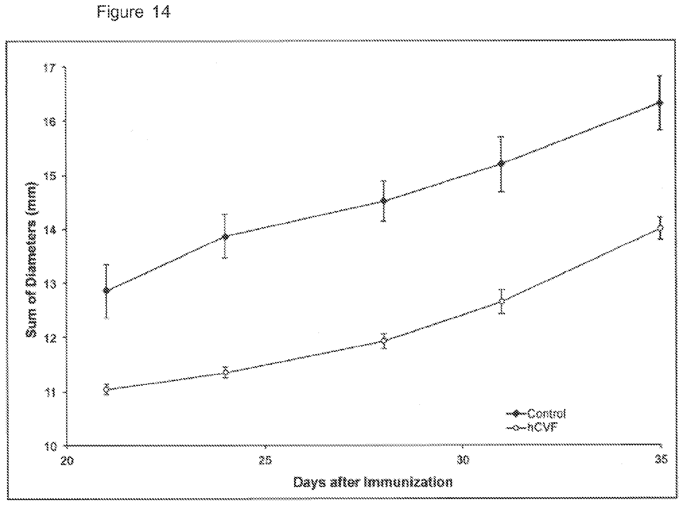

Figure 14. Effect of complement depletion with hCVF in a murine model of collagen-induced arthritis. Shown is the sum of diameters (hind paws, fore paws, and ankles) in mice treated with hCVF starting six days after the booster immunization with collagen.

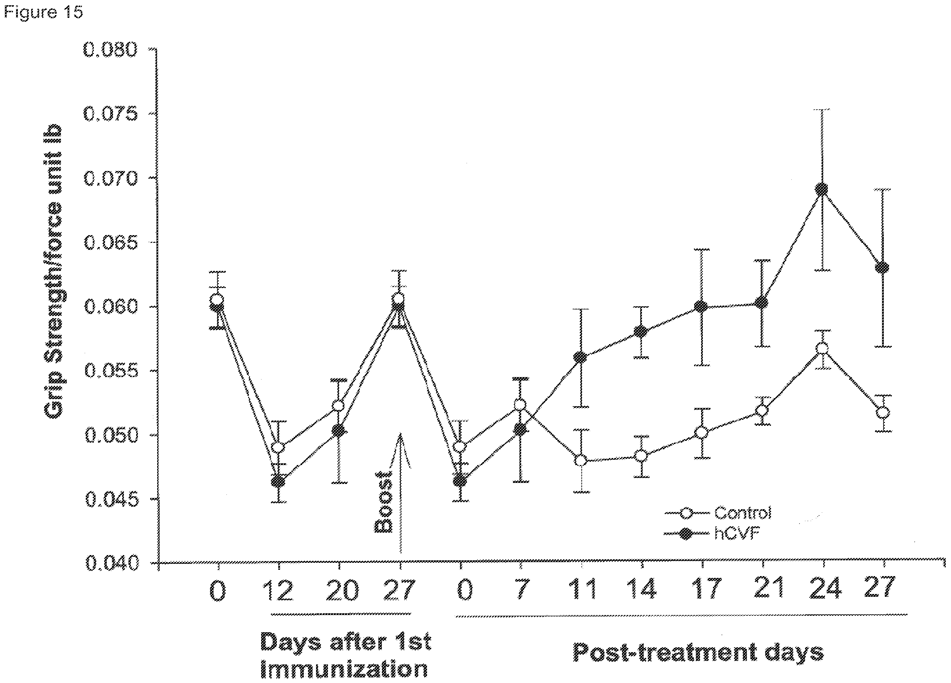

Figure 15. Effective of complement depletion with hCVF in a murine model of experimental autoimmune myasthenia gravis (EAMG). Please note the return of grip strength to pre-immunization levels in animals treated with hCVF.

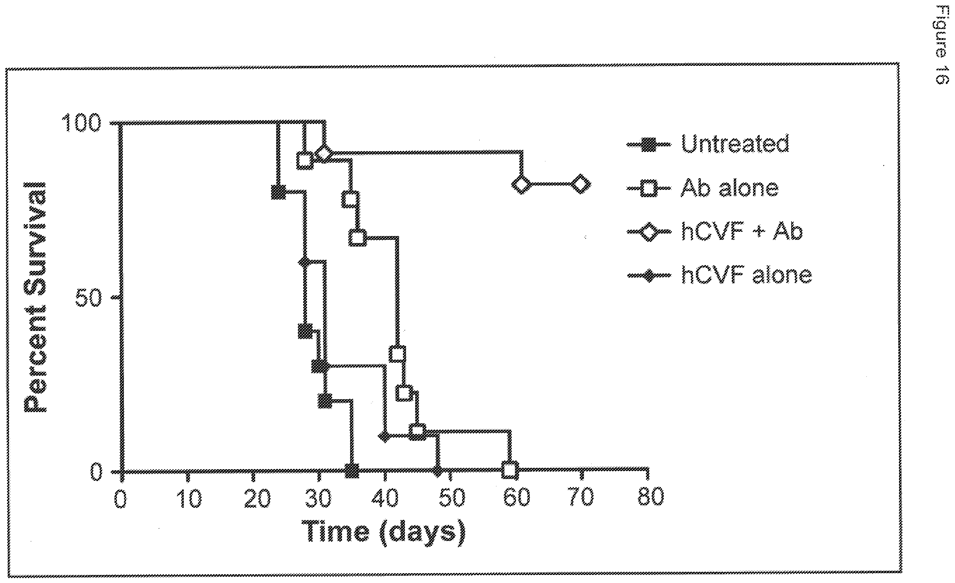

Figure 16. Effect of complement depletion with hCVF on the therapeutic efficacy of monoclonal antibody therapy in a syngeneic genetic mouse model of lymphoma. Please note that complement depletion with hCVF resulted in 80% survival of lymphoma-bearing mice.

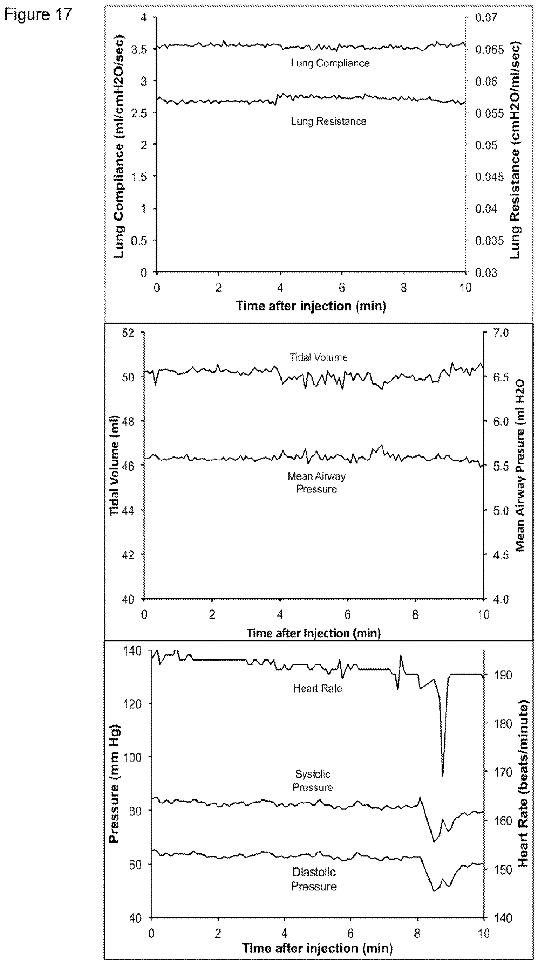

Figure 17. Effect of complement depletion with hCVF on pulmonary and cardiac function in cynomolgus monkey. Complement depletion was induced by intra- arterial injection into the pulmonary artery with hCVF at 250 µg/kg. Shown are physiological lung perimeters, blood pressure, and heart rate as continuously monitored. Note the complete absence of changes in both pulmonary and cardiac function.

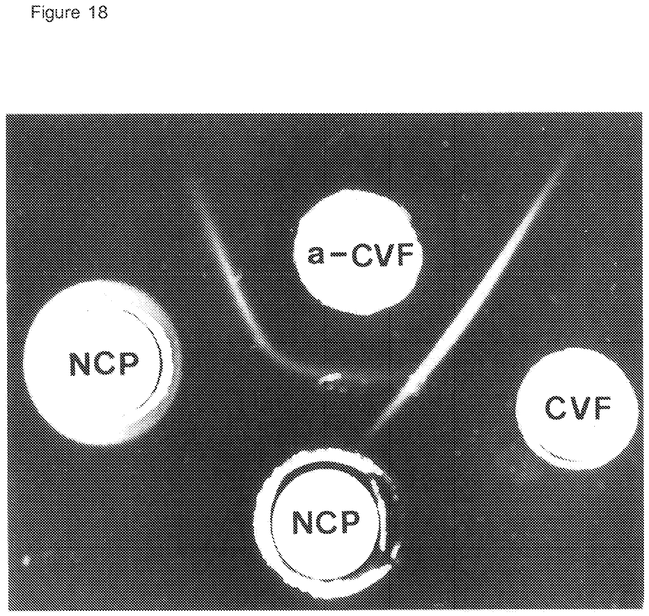

Figure 18. Shown is an Ouchterlony analysis of the immunological cross-reaction of anti-CVF antiserum with cobra C3 in normal cobra plasma (NCP) and purified CVF. Please note the strong cross-reaction of anti-CVF with cobra C3 as well as the dominant spur, indicating the presence of significant antigens on CVF not present on cobra C3.

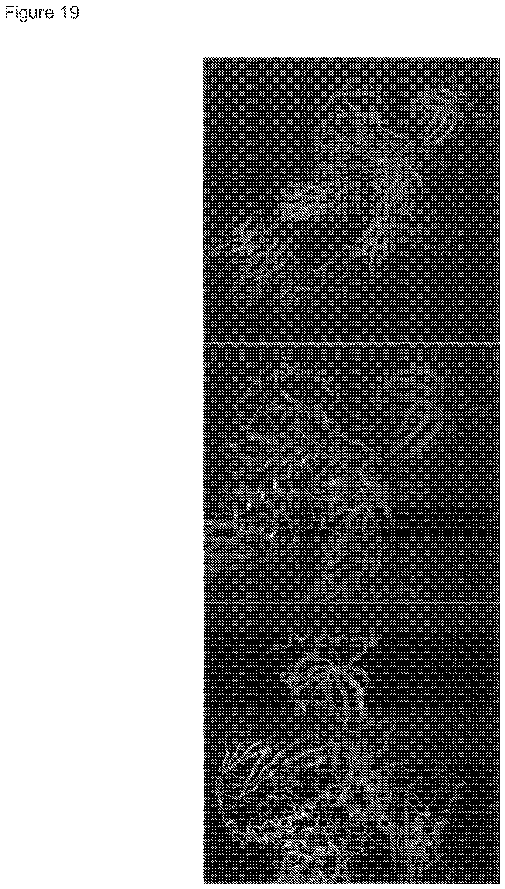

Figure 19. *In silica* prediction of the three-dimensional structure of hCVF proteins HC3-1550 and HC3-1348. The upper panel shows the x-ray crystallographic structure of human C3 superimposed with the predicted structure of HC3-1348. The center panel is a close-up of the C345C and anchor domains from the upper panel. The bottom panel shows the predicted structures of HC3-1498 and HC3-1550 in the C345C and anchor domains simultaneously superimposed with the x- ray crystallographic structure of human C3. Please note the essentially identical structures of all three proteins.

### DETAILED DESCRIPTION

For the convenience of the reader, the following definitions of terms used herein are provided.

The terms "biologic therapy" and "biologic therapeutic" as used herein refer to any biological material suitable for a therapeutic or *in vivo* diagnostic purpose. Biologic therapeutics include peptides, proteins, antibodies, aptamers, nucleic acids, DNA, RNA, antisense oligonucleotides, viruses and bacteria. Biologic therapeutics include medicinal products manufactured in or extracted from biological sources, or engineered from components of living matter such as protein, nucleic acid, carbohydrate, or oligosaccharide, or any combination thereof, including drug therapies, cellular therapies, and biologic medical devices. Exemplary protein biologic therapeutics include, without limitation, growth factors, enzymes, cytokines, peptide hormones, cytokine traps and antibodies.

"Immunogenicity" means the ability of a particular substance to induce a humoral or a cell mediated response of the immune system.

"Unwanted immunogenicity" means when the patient mounts an undesired immune response against a substance such as a therapeutic protein product. Unwanted immunogenicity also refers to the production of antibodies that neutralize biological activities of the therapeutic protein product, which can result in adverse events not only by inhibiting the efficacy of the therapeutic protein product but also by cross-reacting to an endogenous protein counterpart and leading to loss of its physiological function neutralizing antibodies against a therapeutic substance or an immunologically-based adverse event. Immunologically-based adverse events include, without limitation, anaphylaxis, autoimmune disease, cytokine release syndrome, and cross-reactive neutralization of endogenous proteins mediating critical functions.

The invention disclosed herein relates to depleting complement and inhibiting the classical and alternative complement activation pathways. In particular, the invention relates to complement inhibitors derived from cobra venom factor for use in the treatment and prevention of blood clotting disorders associated with immunogenicity against a therapeutic substance. More particularly, disclosed herein are uses of such complement inhibitors to attenuate or inhibit an immune response to a recombinant therapeutic protein or other biologic therapeutic, even when the recombinant therapeutic protein or biologic therapeutic is administered to the patient on a repeated basis. The invention is based at least in part on the discovery that a particular immunomodulatory biologic therapy can prevent unwanted immunogenicity caused by biologic therapies, and that a recombinant protein composed of cobra and human amino acid sequences, which theoretically might be immunogenic, has the opposite and beneficial effect and can prevent unwanted immunogenicity of human and mammalian biologic therapies

Cobra venom contains a structural and functional analog of human Complement 3 (C3) protein called cobra venom factor (CVF). CVF is a complement inhibitor that acts through a mechanism of exhaustive activation which subsequently leads to depletion. CVF is frequently used as the standard to evaluate the anti-complement activity of other drugs. Whereas CVF exhibits this powerful anti-complement activity, it is not suitable for human application because of its immunogenicity. Compositional analysis revealed that CVF contains three *N*-linked oligosaccharide chains per molecule, two in the α-chain and one in the β-chain (Gowda et al., J. Immunol. 152:2977-86 (1994)). While the carbohydrate moieties are not involved in the functions of CVF, it was discovered that oligosaccharide chains of CVF contain unique terminal α-galactosylated Lewis x (Le^{X}) antigenic structures (Gowda et al., Mol. Immunol. 29:335-442 (1992)).

Accordingly, in a first aspect, provided herein is humanized cobra venom factor (hCVF) for use in preventing or attenuating unwanted immunogenicity in a subject resulting from administration of a therapeutic biologic to the subject. According to the invention, an immunomodulator is administered to the subject to reduce or prevent evoking an unwanted immune response upon administration of a biologic therapeutic. The immunomodulatory protein is a humanized cobra venom factor (hCVF) recombinant or fusion protein. Recombinant or fusion humanized cobra venom factor (hCVF) is substantially devoid of immunogenicity but also possesses complement depleting activity.

Any appropriate hCVF recombinant or fusion protein can be used for the invention provided herein. As described herein, hCVF polypeptides suitable for use as immunomodulators are substantially non-immunogenic and substantially devoid of C5-cleaving activity. Exemplary hCVF polypeptides include, without limitation, HC3-1496, HC3-1550b, and HC3-1348. Preferably, the immunomodulator is HC3-1496.

The immunogenicity of preferred embodiments remains low or absent-comparable to that of human C3. As used herein, "substantially non-immunogenic" and "substantially devoid of immunogenicity" mean that hCVF can be from about 75% non-immunogenic to about 100% non-immunogenic, including but not limited to 80%, 85%, 90%, 95%, and 99%.

The hCVF immunomodulator is also a complement depletor. The immunomodulator can be a long-acting or a short-acting complement depletor. For example, some hCVF polypeptides provided herein exhibit short-acting complement-depleting activity but, in some cases, complement levels in a subject to whom the immunomodulator is administered return to pre-administration levels within 12 to 168 hours after administration.

Provided herein are is hCVF for use in treating or preventing a blood clotting disease or condition characterized by or associated with complement pathogenesis. Repeated administration of a biological agent can be associated with evoking an immune response in the subject receiving the biological agent. Prior exposure to a therapeutic protein product or to a structurally similar protein may lead to pre-existing antibodies at baseline. This is a particular concern for patients receiving a replacement product, such as clotting factors or an enzyme replacement therapy, who may have antibodies to a previous product that could cross-react to an analogous product. For example, people who do not have sufficient quantities of coagulation factor VIII (Factor VIII), or whose Factor VIII is defective in some way, suffer from hemophilia A, a disease in which blood clotting is defective and leads to excessive bleeding. Although there is no cure for hemophilia A, infusion of the Factor VIII therapeutic protein can successfully manage the chronic disease. Unfortunately, the development of anti-drug antibodies against the infused Factor VIII is a significant impediment to this strategy. The treatment of patients that develop an immune response is more complex, less effective and exceedingly expensive.

Advantageously, the invention disclosed herein permit repeated (second or subsequent) administrations of biologic therapeutics such as coagulation factor VIII for those subjects that develop or are predicted to develop inhibitory antibodies to the replacement factor. Without being bound by any particular theory, it is believed that the complement-depleting activity of humanized CVF suppresses or prevents the production of antibodies against the biologic therapeutic (e.g., anti-clotting factor antibodies)

Complement depletion according to the invention disclosed herein can be local or systemic. Local treatment may be effected in a number of ways to produce a result of depletion of complement or activation of complement, depending on the desired effect. In one embodiment, local depletion is effected when an immunomodulator is administered locally to an organ, tissue, cavity, or intradermally. This results in a temporary and complete depletion of complement in the area. Alternatively, local activation of complement may employ a specific monoclonal antibody which, when chemically attached to the immunomodulator, can localize it to a specific tissue, a disease, or an infected cell to cause continuous activation of complement in that area. In other embodiments, the antibody can be attached to the immunomodulator via recombinant DNA technology.

Systemic complement depletion is effected when immunomodulators are administered systemically, for example, intravenously or intraperitoneally. This results in a temporary and complete depletion of complement systemically. This method can be used for reperfusion injury, coronary heart surgery, transplantation, and/or systemic disease, particularly during a flare-up or episodical activity.

Any appropriate route of administering humanized CVF can be used according to the invention. In general, intradermal, subcutaneous, and inhalational routes of administration are associated with increased immunogenicity compared to intramuscular and intravenous (i.v.) routes. The intravenous route is generally considered to be the least likely to elicit an immune response.

According to the invention, an immunomodulator provided herein is a biologic agent and is administered to any organism capable of mounting an immune response. In some cases, the organism is a vertebrate such as a mammal (e.g., primate) or bird. Exemplary organisms for the compositions and methods described herein include without limitation human and non-human primates, domestic animals, livestock, and birds.

### EXAMPLES

### Example 1 -- Inhibition of Immunogenicity to Therapeutic Biologic by Humanized Snake Venom Fusion Protein

Four doses of humanized cobra venom factor fusion protein, HC3-1496, was injected, each dose before the administration of therapeutic Factor VIII to Factor VIII-deficient mice, once a week for 4 weeks. No unwanted immunogenic reaction resulted over 4 weeks as measured by a significant decrease in anti-Factor VIII IgG and inhibitor titers as compared to control mice (p<0.0001). Moreover, no unwanted immunogenic reaction results from the administration of HC3-1496.

### Example 2 -- Contribution of Complement to the Immune Response Against Therapeutic FVIII in Hemophilia A

**Introduction.** Hemophilia A is a hemorrhagic disease due to a deficiency in coagulation factor (FVIII). Replacement therapy consists in the administration of therapeutic FVIII. In 5 to 30% of the cases, FVIII injections result in the emergence of anti-FVIII antibodies that inhibit the pro-coagulant activity of therapeutic FVIII.

**Objective.** We studied the contribution of complement to the immune response against therapeutic FVIII in hemophilia A.

**Methods.** Complement was depleted in FVIII-deficient mice using humanized cobra venom factor before the administration of therapeutic FVIII, once a week for 4 weeks. Anti-FVIII IgG and inhibitory titers were measured by ELISA and FVIII pro-coagulant chromogenic assay, respectively. Complement effect on FVIII endocytosis by immature monocyte-derived DCs (iMo- DCs) and FVIII presentation to a FVIII-specific T-cell hybridoma were studied.

**Results.** Complement depletion in FVIII-deficient mice resulted in a significant decrease in anti-FVIII IgG and inhibitor titers as compared to control mice (P<0.0001). Complement depletion was validated by the measure of C3 in serum. FVIII was found to bind C1q. Our results show that complement contributes to FVIII endocytosis by iMo-DCs. The endocytosis was reduced in C1q and C3-depleted serum as compared to normal serum (P=0.006 and P=0.009, respectively). FVIII endocytosis correlated with FVIII presentation to a FVIII-specific T-cell hybridoma. Moreover, pre-incubation of FVIII with C1q enhanced FVIII presentation to CD4+ T cells in a dose-dependent manner.

**Conclusion.** Our results show that complement depletion using humanized cobra venom factor decreases the immune response against therapeutic FVIII. Moreover, FVIII opsonisation by C1q and C3 enhances the endocytosis of FVIII by iMo-DCs and its presentation to CD4+ T lymphocytes.

### Example 3 -- Humanized Cobra Venom Factor Exhibits Virtual Absence of Immunogenicity in Mice Compared to Natural CVF

In a murine model of hemophilia A (Factor VIII knock-out mice), we used cobra venom factor (CVF) to deplete complement in order to investigate the role of complement in the immune response to Factor VIII, a clinically relevant problem in up to 30% of hemophilia patients. We used weekly injections of CVF. Whereas the first injection of CVF resulted, as expected, in essentially complete elimination of serum C3, starting with the second injection the reduction of serum C3 levels was significantly impaired. At week 4, CVF injection resulted in moderate C3 reduction in only two mice and was without effect in the majority of the mice. We subsequently used humanized CVF (hCVF) protein HC3-1496, a human C3 derivative with CVF-like function for complement depletion. HC3-1496 exhibits 94% sequence identity and 96% sequence similarity with human C3. hCVF resulted in effective reduction of serum C3 even at week 4, suggesting the absence of any functionally relevant immune response. The lower immunogenicity of hCVF in mice is likely a consequence of the higher amino acid sequence identity between murine C3 and human C3 (77%) compared to CVF (52%). Moreover, the recombinantly produced hCVF lacks the unusual cobra oligosaccharide chains of CVF which likely are immunogenic. In line with this, we expect that the immunogenicity of hCVF is reduced in humans.

We have developed a distinctly different therapeutic approach: complement depletion rather than inhibition. This approach is based on cobra venom factor (CVF), a C3 analog known to be able to safely deplete complement. Exploiting the knowledge of the structure/function relationship of CVF and C3, we created derivatives of human C3 which display the CVF-like activity of depleting complement, referred to as humanized CVF (hCVF). In the following sections, we described the structure and activity of hCVF, including the important property of not cleaving C5.

The efficacy of hCVF for therapeutic complement depletion in nine preclinical models diseases with complement pathology is reviewed, including reperfusion injury, age- related macular degeneration (AMD), paroxysmal nocturnal hemoglobinuria (PNH), and immunogenicity of Factor VIII in hemophilia A. Complement depletion is characterized by the absence of toxicity, even after intra-arterial injection into the pulmonary artery of primates. No immunogenicity has been observed.

Abbreviations used: CVF, cobra venom factor; rCVF, recombinant CVF; hCVF, humanized CVF; MAC; membrane attack complex; PNH, paroxysmal nocturnal hemoglobinuria; MI/RI, myocardial ischemia reperfusion injury; GI/RI, gastrointestinal ischemia reperfusion injury; AMD, age-related macular degeneration; EAMG, experimental autoimmune myasthenia gravis; AChR, acetylcholine receptor; aHUS, atypical hemolytic uremic syndrome; NCP, normal cobra plasma.

Information about Convention on International Trade in Endangered Species of Wild Fauna and Flora (CITES) is available on the World Wide Web at cities.org.

Nomenclature of hCVF proteins: The number given in the name of the hCVF protein (e.g., HC3-1496) is the first amino acid residue replaced by CVF sequence, using human C3 pre-pro-protein numbering.

### 1. Introduction

The complement system is part of the immune system and has important functions in both innate and adaptive immunity. However, the complement system also plays an important role in the pathogenesis of many diseases, contributing solely or significantly to the disease process and tissue injury. Table 1 lists selected diseases with confirmed complement pathogenesis. Because of its role in the pathogenesis of many diseases, the last two decades have seen multiple approaches to developing pharmacological agents to interfere with or modulate the complement cascade. These anti- complement agents, small and large, can be grouped into two conceptually different categories. One group of inhibitors is aimed at a specific complement component and will inhibit its activation. Examples include a humanized antibody to C5 which will prevent its activation and therefore prevent the formation of the membrane attack complex (MAC) and ensuing tissue damage, approved for treatment of paroxysmal nocturnal hemoglobinuria (PNH) and atypical hemolytic uremic syndrome (aHUS). Another example of a complement inhibitor is compstatin, a cyclic β- residues peptide that binds to C3 and prevents its subsequent activation. A second group of complement inhibitors is represented by agents that do not prevent the activation of a complement component but inhibit the action of an activated complement component. Examples include antagonist for the C5a receptor designed to inhibit the pro-inflammatory activities of C5a. Another example is a chimeric protein consisting of a regulatory domain of human Factor H (CCP1-5) with a C3d-binding domain from complement receptor 2 (CR2) (CCP1-4). This chimeric protein targets sites of ongoing complement activation and will cause the inhibition of further complement activation by inactivating C3b. These approaches have shown encouraging results in multiple disease models with complement pathogenesis.

We have developed a third, distinct approach that is neither based on inhibition of a complement component nor its activated fragment but on depletion of complement. Our concept is based on cobra venom factor (CVF), an unusual venom component of cobras. As described below, CVF is a structural and functional analog of complement component C3 that forms a stable C3/C5 convertase and exhibits resistance to the regulatory complement proteins, leading to exhausted complement activation and, therefore, complement depletion. Ever since it had been shown that CVF can be safely administered to laboratory animals for complement depletion, CVF has become an important research tool to study the role of complement in both its biological functions as well as in the pathogenesis of disease. By comparing normal (complement-sufficient) animals with complement-depleted animals, the involvement of complement in physiological and pathological situations could be elucidated. Moreover, complement involvement in the pathogenesis of many diseases was first recognized by using animals depleted of their complement with CVF. CVF has also served as the gold standard to compare the efficacy of other complement inhibitors. Herein, we describe the structure and function of CVF and its homology to complement component C3 as well as our work to create human C3 derivatives with the complement-depleting function of CVF (collectively referred to as humanized CVF (hCVF)) as a novel therapeutic approach for treatment of diseases and clinical conditions with complement pathogenesis.

### 2. Cobra venom factor

CVF is the complement-activating protein in the venom of cobras. Whereas more recent work has established that probably all members of the *Naja* genus and other elapid snakes (e.g., *Ophiophagus, Austrelaps)* produce CVF in their venom, the majority of previous work is based on CVF isolated from the Indian cobra *(Naja naja)* or a closely related Asian cobra species *(Naja kaouthia).* CVF is a structural and functional analog of complement components C3.

Figure 1 shows the schematic chain structures of C3, C3b, C3c, and CVF. CVF exhibits a high degree of sequence homology at both the DNA and protein level with C3 from mammalian and other vertebrate species; and its homology exceeds 90% with C3 from cobra. Like C3, CVF is synthesized as a single-chain pre-pro-protein that is subsequently processed into the mature three-chain protein. The genes for C3 and CVF exhibit a high degree of homology with an essentially identical exon/intron structure. The homology of the three chains of CVF with the corresponding chains of C3 has not only been established by amino acid homology and immunological cross reactivity but more recently by the crystal structures. The three-dimensional structures of C3 and CVF display identical domains. Whereas the three-chain CVF resembles the physiological three-chain degradation product C3c, it differs in one important aspect. In contrast to C3c, CVF contains the intact CUB domain which has been shown to be important for Factor B binding and convertase formation (Figure 2). CVF, a glycoprotein, has three glycosylation sites for N-linked oligosaccharides chains, two of which are in the α-chain and one is in the β -chain. The overall carbohydrate content of CVF is 7.4% (w/w), which is significantly higher than that of human C3 (1.7%) and other mammalian C3 species. We have extensively characterized the structures of the CVF carbohydrate chains, with the major oligosaccharide chain being a symmetric fucosylated biantennary complex-type chain with an unusual α-galactosylated Le^{x} structure at its non-reducing end (Figure 3). However, the glycosylation of CVF is not required for its biological activity as partial or complete deglycosylation as well as the introduction of charges into the carbohydrate chains has no functional consequences. However, the antigenically unique oligosaccharides chains may likely contribute to the immunogenicity of CVF (see Section 4.3 below).

The extensive structural homology between CVF and C3 is paralleled by a functional homology. Like C3b, CVF binds Factor Bin the presence of Mg²⁺ ions. Just like the C3b,B complex, the CVF,B complex is the substrate for Factor D which cleaves Factor B, releasing the activation peptide Ba and generating the bimolecular complex CVF,Bb. Like C3b,Bb, CVF,Bb is a C3 convertase cleaving C3 into C3a and C3b. Furthermore, like C3b,Bb, CVF,Bb is also a CS convertase cleaving CS into C5a and C5b. Accordingly, both enzymes, C3b,Bb and CVF,Bb, are referred to as C3/C5 convertases of the alternative complement pathway, and are given a single EC number (EC 3.4.21.47). In both convertases, the enzymatically active site resides in the identical Bb subunit of the bimolecular enzyme.

Although both enzymes share the molecular architecture, consisting of a structural subunit (CVF or C3b) and the identical active site-bearing subunit Bb as well the substrate specificities for C3 and C5, the two enzymes exhibit significant functional differences. First, although both enzymes are intrinsically unstable, exhibiting spontaneous decay-dissociation into the respective subunits, which abolishes the enzymatic activity, the physicochemical half-lives of the C3b,Bb and CVF,Bb enzymes differ significantly. Whereas the C3b,Bb enzyme is extremely unstable and dissociates with a half-life of approximately 1.5 minutes at 37°C, the CVF,Bb enzyme is very stable. Its half-life of decay-dissociation at 37°C is approximately seven hours.

Second, the C3b,Bb convertase is subject to rapid and efficient inactivation by the complement regulatory proteins Factors H and I. Factor H dissociates C3b,Bb and serves as cofactor for the proteolytic inactivation of C3b by Factor I. In contrast, both the CVF,Bb convertase and CVF are completely resistant to the regulatory actions of Factors H and I.

Third, although both enzymes can cleave C5, fluid-phase C3b,Bb is essentially devoid of C5-cleaving activity because the Km of the monomeric C3b,Bb enzyme for C5 (24 µm) *is* well above the physiological C5 concentration in plasma (0.37 µm). In contrast to C3b,Bb, CVF,Bb exhibits fluid-phase C5-cleaving activity, consistent with a Km value of 0.036 µm, well below the physiological C5 concentration in plasma.

The physicochemical stability of CVF,Bb and its resistance to inactivation by Factors H and **I** is the molecular basis for its ability to deplete serum complement, both *in vitro* and *in vivo.* When CVF is added to serum, the CVF,Bb enzyme forms and will continuously cleave both C3 and C5. C5b, the activated form of C5, will also consume the complement components of the terminal membrane attack pathway. As a consequence of the continuous action on C3 and C5, the CVF,Bb enzyme will lead to depletion of serum complement, although the functional depletion of serum complement activity is primarily a function of C3 cleavage and not C5 cleavage. Figure 4 demonstrates complement depletion by CVF in mice. After a single dose of CVF, plasma complement activity is rapidly depleted to very low levels, and returns to normal levels within seven to five days.

### 3. Humanized Cobra Venom Factor

As much as CVF represents a tool for experimental complement depletion in laboratory animals as described above, depletion of complement also represents a therapeutic concept for treatment of diseases with complement pathogenesis. Eliminating complement from the plasma will prevent harmful effect of complement, independent of the presence of a complement-activating trigger.

For several reasons CVF itself would not be a suitable drug candidate. For one, its natural source, cobra venom, is obviously of limited supply. Moreover, most species of the genus *Naja* are protected by the Convention on International Trade in Endangered Species (CITES). Lastly, CVF, a protein from cobra, is highly immunogenic in mammals. The development of a clinically useful agent for therapeutic complement depletion would therefore entail both recombinant production and significantly reduced immunogenicity. Both aims have been achieved with the generation of humanized CVF (hCVF) as described below.

After successfully cloning CVF, we were subsequently able to express recombinant forms of CVF (rCVF). Using the baculovirus *Spodoptera frugiperda* Sf9 cells, rCVF was expressed as a single-chain pro-protein resembling pro-C3. More recently we used stably transformed *Drosophila* S2 cells for expression of rCVF. Using S2 cells, the four arginine residues separating the α- and β-chain equivalents of CVF are removed, and the C3a-like domain is removed to a varying degree. Accordingly, S2 cell-expressed rCVF is a mixture of two two- chain forms resembling C3 and C3b (comp. Figure 1). No processing to the three-chain form of natural CVF occurred. Surprisingly, all three forms of rCVF exhibit CVF activity indistinguishable from natural CVF: rCVF forms a stable convertase with Factor B, exhibiting both C3-cleavage and C5-cleavage as well as complement-depleting activity like CVF,Bb.

The ability to recombinantly produce active CVF represents not only an opportunity to produce large quantities for potential clinical applications. It also is a tool to study the structure/function relationship of CVF and C3, including immunogenicity. By taking advantage of the high degree of homology between C3 and CVF, we created hybrid or chimeric proteins of the two proteins. Our initial goal was to identify the crucial structures in CVF responsible for its functional difference to C3, namely forming a physicochemically stable convertase and being resistant to Factors H and I. Our first approach was to generate loss-of-function-hybrids by exchanging portions of CVF with cobra C3. This work showed that the C-terminal region of the CVF β-chain (equivalent to the C3 α-chain) harbors the crucial structures for forming a stable convertase, thereby confirming our earlier results from limited proteolysis of CVF that showed that removal of the N-terminal portion of the β-chain did not abolish activity.

Once the crucial region in the CVF molecule was identified, the next step was to create gain-of-function hybrids in which the C-terminal end of the C3 a-chain in human C3 was replaced with homologous sequences from CVF. We found that the introduction of CVF sequences at the C-terminal portion of the human C3 α-chain created indeed molecules that exhibited properties of CVF. They are human C3 derivatives, and are referred to as humanized CVF (hCVF). Figure 5 shows the schematic chain structures of hCVF proteins mentioned here. Production of recombinant hCVF proteins in S2 cells resulted in a mixture of C3-like and C3b-like forms just like rCVF (Figure 6). More recently, we also used stably transformed Chinese hamster ovary (CHO) cells for expression which resulted in more homogeneous hCVF proteins (Figure 6) and allowed the production in gram quantities. hCVF proteins form stable convertases with human Factor B, with several hCVF,Bb convertases exhibiting a stability resembling or even exceeding that of CVF,Bb (Table 2). Similarly, convertases formed with hCVF proteins exhibit C3-cleaving activity resembling or exceeding that of CVF,Bb (Figure 7A). Humanized CVF proteins also display complement-depleting activity in serum *in vitro* (Figure 7B). The complement-depleting activity of hCVF was entirely unexpected. Independent of how stable a hCVF convertase with Factor B would be, hCVF contains all known binding sites for Factor H and all three cleavage sites for Factor I within its C3 portion, and would have been expected to be rapidly inactivated by Factors H and I, preventing complement-depleting activity in serum. As it turns out, the C-terminal region of the CVF β-chain confers not only the property of being able to form a stable convertase with Factor B but also of partial resistance to inactivation by Factor H and I (Figure 7C), a fortuitous property for clinical application.

Another property of hCVF, although equally fortuitous for clinical application, is the fact that convertases formed with hCVF do not cleave C5 and therefore do not generate C5a (Figure 8). This property of hCVF was entirely unexpected as both natural CVF and rCVF as well as, at least under certain conditions, human C3b form convertases exhibiting C5-cleaving activity. The molecular basis for C5 cleavage by convertases, or lack of, is not understood.

Whereas hCVF proteins deplete serum complement *in vivo* as quickly and efficiently as CVF, the time period of complement depletion is not as pronounced, with complement levels regaining pre-depletion levels within 24 to 48 hours, with some species to species variation (Figure 9). The shorter complement depletion by hCVF is most likely a consequence of the fact that hCVF only displays partial resistance to inactivation by Factors H and I (compare to Figure 7C) which would lead to removal of inactivated hCVF protein and will prevent, in contrast to CVF, reformation of new convertase molecules.

We created a large number of chimeric human C3/CVF proteins. Different chimeric proteins differ tremendously in their properties of convertase stability, C3 cleavage, and complement-depletion; and we have shown that small changes of just a few amino acid residues can have a major effect on activities. These chimeric proteins are not only valuable tools to study the structure and function of C3 and CVF but offer the opportunity to create new hCVF proteins with further improved properties.

### 4. Humanized CVF for therapeutic complement depletion in preclinical models of disease

We have chosen the hCVF protein HC3-1496 for our preclinical evaluation of therapeutic complement depletion. Below, we will review the efficacy of complement depletion with hCVF protein HC3-1496 in multiple preclinical models of human diseases.

### 4.1. Efficacy of humanized CVF in preclinical disease models

### 4.1.1 Age-related macular degeneration (AMD) (not part of the invention)

We used a murine model of age-related macular degeneration (AMD) based on laser-induced photocoagulation of the Bruch's membrane and choroidal neovascularization. Complement-depletion was accomplished by daily i.p. injection of HC3-1496 at a rather low dose (25 µg/kg) prior to and daily after laser surgery for 28 days. Figure 10 shows that complement depletion with HC3-1496 resulted in smaller lesions as demonstrated by both, funduscopic images after i.v. injection of FITC-dextran on days 0 and 8 as well as histopathological examination of retinal lesions in formalin-fixed eyes on day 28. Similar results were obtained in CVF transgenic mice which constitutively express CVF and exhibit low serum C3 levels and low complement activity.

### 4.1.2. Gastrointestinal ischemia reperfusion injury (GIIRI) (not part of the invention)

We used a murine model of gastrointestinal ischemia reperfusion injury (GI/RI) to assess the effect of complement-depletion with hCVF protein HC3-1496. In this model, intestinal ischemia was produced for 20 minutes by occlusion of the superior mesenteric artery, followed by three hours of the perfusion. Complement depletion was accomplished by i.p. injection of HC3-1496 at 250 µg/kg two hours prior to anesthesia. Intestinal tissue damage was assessed by measuring FITC-conjugated dextran in serum after luminal enteral administration. As shown in Figure 11, decomplementation with hCVF protein HC3-1496 resulted in a significantly reduced reperfusion injury.

### 4.1.3. Myocardial ischemia reperfusion injury (MI/RI) (not part of the invention)

We used a murine model of myocardial ischemia reperfusion injury (MI/RI) to assess the effect of complement depletion with hCVF protein HC3-1496. In this model, myocardial ischemia was induced by suture of the left anterior descending coronary artery. After 30 minutes of the ischemia, the myocardium was reperfused for four hours. As shown in Figure 12, decomplementation with hCVF protein HC3-1496 at 250 µg/kg two hours prior to the induction of in anesthesia resulted in a significant reduction of the reperfusion injury as demonstrated immunohistochemically by reduced deposition of C3b and morphologically by a smaller infarct size, concomitant with improved preservation of myocardial function as shown by both fractional shortening and injection fraction.

### 4.1.4. Ventilator-induced lung injury (VILI) (not part of the invention)

In a murine model of ventilator-induced lung injury (VILI), we assessed the effect of complement-depletion with hCVF protein HC3-1496. Complement-depletion with hCVF at 250 µg/kg significantly reduced lung damage as measured by reduced deposition of C3 (Figure 13) as well as an increased number of single cells bronchoalveolar lavage fluid.

### 4.1.5. Arthritis (not part of the invention)

We used a murine model of collagen-induced arthritis to assess the effect of complement depletion with hCVF protein HC3-1496. In this model, arthritis was induced by immunization with collagen. Complement depletion was achieved with an i.p. injection of hCVF protein HC3-1496 at 500 µg/kg six days after the booster immunization with collagen, and subsequently with a maintenance dose of 250 µg/kg administered at five days per week for three weeks. Arthritis was monitored by measuring the diameters of hind paws, fore paws, and ankles. As shown in Figure 14, complement depletion resulted in signification reduction of the gross pathological changes and a concomitant reduction of the swellings.

### 4.1.6. Paroxysmal nocturnal hemoglobinuria (PNH)

Paroxysmal nocturnal hemoglobinuria (PNH) is a relatively rare but potentially life-threatening disease in which a defect in the glycosylphosphatidylinositol (GPI) anchor results in the absence of the protective regulatory proteins CD55 and CD59 on the surface of red blood cells. In our model, we used PNH red blood cells from human patients incubated in the presence of a recombinantly produced truncated form of Factor H (rH19-20) which makes PNH cells highly susceptible to complement lysis in normal human serum (NHS). As shown in Table 3, decomplementation of human serum by hCVF or CVF protected PNH cells from complement lysis.

### 4.1.7. Hemophilia A

Up to 30% of hemophilia A patients develop antibodies against recombinant human Factor VIII. We used mice deficient in Factor VIII to assess the effect of complement depletion with hCVF protein HC3-1496 on the production on anti-Factor VIII antibodies. Complement-depletion was achieved by i.p. injection of hCVF prior to i.v. injection of human recombinant Factor VIII once per week for four weeks. We found that complement depletion with hCVF resulted in a significant reduction of anti-Factor VIII antibody titers.

### 4.1.8. Myasthenia gravis (not part of the invention)

Experimental autoimmune immune myasthenia gravis (EAMG) is a mouse model of myasthenia gravis, an autoimmune disease of unknown etiology characterized by the occurrence of auto-antibodies against the acetylcholine receptors (AChR) in the neuromuscular junctions. The symptoms of muscle weakness and paralysis are produced by complement-mediated destruction of AChR. We used EAMG to assess the effect of the complement depletion with hCVF protein HC3-1496. Mice were immunized with affinity-purified AChR from *Torpedo* with an initial immunization and a booster immunization two weeks later. Two weeks after the booster immunization, mice were complement-depleted by daily i.p. injections for 30 days with HC3-1496 at 500 µg/kg. As shown in Figure 15, grip strength dropped significantly after the initial immunization and the booster immunization. Whereas grip strength remained low in untreated animals, animals complement-depleted with HC3-1496 regained normal grip strength within two to three weeks. The better muscular function of hCVF-treated mice corroborated with the presence of more AChR and a virtual lack of membrane attack complexes (MAC) at the neuromuscular junctions.

### 4.1.9. Monoclonal antibody therapy of lymphoma (not part of the invention)

Monoclonal antibodies against CD20 such as rituximab® are widely used in the treatment of 8-celllymphomas. Several studies have shown that both complement activation and antibody-dependent cellular cytotoxicity (ADCC) may contribute to the anti-tumor activity. However, complement activation my actually interfere with the binding of NK cells to rituximab-coated tumor cells, thereby inhibiting NK cell-mediated lysis. We found that complement depletion with hCVF protein HC3-1496 prevents the inhibitory effect of complement on NK cell activation *in vitro.* Using a murine model of lymphoma (38C13 lymphoma cells; MF11G6 anti-lymphoma monoclonal antibody), we found that mice decomplemented i.p. with HC3-1496 at 400 µg/kg three days after tumor cell inoculation and four hours prior to antibody injection, followed by another dose of HC3-1496 two days later, resulted in an 80% survival (Figure 16). This is a stark contrast to untreated mice and mice treated with either the antibody or HC3-1496 alone (Figure 16). We subsequently used a Raji cell mouse lymphoma model with various combination therapies of rituximab® and HC3-1496. Although not as pronounced, a therapeutic effect of complement depletion was observed, with 25% survival of up to 115 days.

### 4.2. Lack of toxicity of complement depletion with humanized CVF

There are three conceptually different ways that complement depletion could exhibit harmful effects. Acute side effects could develop as a consequence of the rapid process of fluid-phase complement activation. More long-term side effects could be a consequence of the state of being complement-depleted. Lastly, CVF and hCVF could exhibit off-target toxicity not related to complement activation. However, given the protein nature of CVF and hCVF and their extremely high binding specificity for Factor B, any off-target side effect by CVF or hCVF are very unlikely and, indeed, have never been observed.

The only known side effect of massive fluid-phase complement activation by natural CVF is a consequence of the generated anaphylatoxins C3a and C5a. Both anaphylatoxins are readily inactivated by carboxypeptidase N to C3a-des- Arg and C5a-des-Arg, respectively. However, C5a-des-Arg retains its ability to activate neutrophils, which have been shown to be sequestered in the lungs, causing acute but fleeting inflammatory lung injury. Despite well over 40 years of use of CVF in experimental animals, no other acute side effect has been observed.

In contrast to natural CVF, hCVF lacks C5-cleaving activity and does not generate C5a (comp. Figure 8), and no lung damage would therefore be expected from complement depletion with hCVF. Indeed, as shown in Figure 17, no effect on pulmonary function was observed in cynomolgus monkeys even though complement depletion was achieved by intra-arterial administration of hCVF protein HC3-1496 at 250 µg/kg or even 1,000 µg/kg into the pulmonary artery. Similarly, no effect on cardiac function was observed at the dose of 250 µg/kg (Figure 17). At the relatively high dose of 1,000 µg/kg, also directly injected into the pulmonary artery, a temporary increase in systolic blood pressure and a moderate increase in the heart rate were observed, although it remains to be determined if these effects were a consequence of the complement activation. Consistent with the long use of CVF for complement depletion in laboratory animals, no acute toxic side effects were observed in any of the above-described preclinical models of complement depletion with hCVF.

Other adverse side effects could be a consequence of being in a state of prolonged complement depletion. Homozygous genetic deficiency of virtually all complement components has been described in humans and animals. The clinical phenotype for deficiencies of the early components of the classical pathway is a lupus-like autoimmune disease, recurrent infectious meningitis with gram-negative bacteria is the hall mark of deficiencies in terminal membrane attacked pathway components, and recurrent infections with gram-positive bacteria occur in C3 deficiency, the clinically most serious genetic deficiency of a complement component. Similarly, C3 knockout mice show an increased susceptibility to infections but no other pathology.

In contrast to genetic deficiencies, complement depletion with CVF or hCVF has not shown any adverse effect from long-term depletion, although depletion with CVF is more or less limited to no more than two to three weeks because of its immunogenicity (see Section 4.3., below)and hCVF has so far only been used for depletion of up to one month. Significantly, CVF transgenic mice, constitutively expressing CVF and living with low C3 levels and low serum complement activity exhibit a normal life span and no abnormal phenotype. No tendency to develop infections was observed although the CVF transgenic mice were kept under normal animal housing condition. The important difference between genetic C3 deficiency and long-term complement depletion in CVF transgenic mice is the fact that C3 is never completely depleted in the latter case. This is the consequence of the fact that the C3 convertase CVF,Bb obeys Michaelis-Menton kinetics; and as the C3 concentration in plasma decreases as a consequence of enzyme action, the rate of C3 conversion slows down as the C3 concentration drops way below the Km of the enzyme. Apparently, a residual C3 concentration is sufficient to prevent serious consequences of long-term complement-depletion.

### 4.3. Potential immunogenicity of humanized CVF

CVF is a protein from a reptile and as such, given the phylogenetic distance, immunogenic in mammals. Due to its immunogenicity, its usefulness for complement depletion in mammals is essentially limited to a single injection. Given the overall three-dimensional structural similarity of CVF and C3, along with a protein sequence identity of over 50% between CVF mammalian C3s, it is not surprising that anti-sera to CVF have been shown to exhibit some weak cross-reactivity with human C3. In contrast, anti-CVF exhibits a stronger cross-reaction with cobra C3, given a protein sequence identity of over 85% (Figure 18). There is good circumstantial evidence that the oligosaccharide chains of CVF significantly contribute to its immunogenicity. As mentioned above, the CVF oligosaccharide chains contain unusual sugar structures. Ouchterlony analysis of the cross-reaction between CVF and cobra C3 demonstrates a strong spur formation (Figure 18), certainly suggesting a major contribution from carbohydrate epitopes. This assumption is corroborated by the fact that cobra C3 is not glycosylated and that among monoclonal antibodies raised against CVF there were several were specificity for carbohydrate antigens.

Collectively, this knowledge, along with the fact the oligosaccharide chains of CVF are not required for its function, was the basis for our approach to develop a humanized version of CVF with significantly reduced or potentially absent immunogenicity. The concept was to generate derivatives of humans C3 with CVF-Iike function by introducing relatively short amino acid sequences from CVF. This approach, as described above, was successful. In the case of hCVF protein HC3-1496, the protein sequence identity between human C3 and HC3-1496 is 94%, with a sequence similarity of 96%. Significantly, even within the 168 C-terminal residues from CVF, 44% are identical to human C3, and 64% are similar. Moreover, recombinant production of hCVF in eukaryotic cells such as insect or hamster cells results in glycosylation that is more similar to human. Additional support for low immunogenicity of hCVF stems from an *in silica* prediction of the hCVF three-dimensional structure in the C345C region using homology modeling software which shows essentially identical structures to human C3 (Figure 19).

Ultimately, the immunogenicity of hCVF in humans cannot be predicted. However, the prediction of low immunogenicity of hCVF is supported by our finding that complement-depletion with hCVF of up to 30 days was effective in several of our preclinical models as described above (AMD, arthritis, myasthenia gravis, hemophilia A). Importantly, our recent results in the murine model of hemophilia A demonstrated that complement depletion with natural CVF was essentially ineffective after four weeks of weekly injections. In contrast, hCVF resulted in efficient complement-depletion even after the fourth weekly administration. Given the higher sequence identity of HC3-1496 to human C3 (94%) compared to murine C3 (74%), it is reasonable to expect that hCVF protein HC3-1496 will be even less immunogenic in humans.

### 5. Conclusions

hCVF represents a conceptually different and promising therapeutic agent for complement depletion in diseases with complement pathology. Given the very encouraging results of complement depletion with hCVF in numerous preclinical studies, it appears reasonable to predict that complement depletion with hCVF will become an effective clinical tool. hCVF protein HC3-1496 is suited for clinical situations requiring short-term complement depletion, even on a repeated basis. For clinical applications requiring longer-term complement depletion, new hCVF proteins causing prolonged complement depletion will need to be developed.

**Table 1. Diseases with complement pathogenesis**

| **Disease** |
|---|
| Rheumatoid arthritis (not part of the invention) |
| Lupus erythematosus (not part of the invention) |
| Myasthenia gravis (not part of the invention) |
| Hyperacute rejection after xenotransplantation (not part of the invention) |
| Age-related macular degeneration (AMD) (not part of the invention) |
| lschemia/reperfusion injury (not part of the invention) |
| Paroxysmal nocturnal hemoglobinuria (PNH) |
| Bullous pemphigoid (not part of the invention) |
| Asthma (not part of the invention) |
| Anti-phospholipid syndrome (not part of the invention) |
| Atypical hemolytic uremic syndrome (aHUS) |

**Table 2. Physicochemical stability of convertases formed with hCVF proteins**

| **Convertase** | **Half-life at 25°C (hrs)¹** |
|---|---|
| C3b,Bb | 0.072 (4.3 min) |
| HC3-1550b,Bb | 2.0 |
| HC3-1348,Bb | 28.7 |
| HC3-1496,Bb | 31.2 |
| CVF,Bb | 19.2 |

| | |
|---|---|
| ¹Half-life of the spontaneous decay-dissociation as determined by surface plasmon resonance | |

**Table 3. Lysis of PNH cells by human serum depleted with hCVF protein HC3-1496 or natural CVF**

| Treatment | % Hemolytic activity | %Cells lysed |
|---|---|---|
| None | 100% | 84% |
| hCVF | 17% | 5% |
| CVF | 14% | 0% |

## Claims

1. Humanized cobra venom factor (hCVF) for use in reducing or preventing an immune response to a clotting factor in a subject to whom the clotting factor is administered, wherein a therapeutically effective amount of humanized cobra venom factor (hCVF) and said clotting factor is administered to the subject, whereby administration of hCVF depletes complement levels in blood of the subject.

2. hCVF for use according to claim 1, wherein administration of the clotting factor and hCVF is a second or a subsequent administration to the subject and the immune response is reduced or prevented by said second or subsequent administration.

3. hCVF for use according to claim 1, wherein hCVF is administered prior to administration of the clotting factor.

4. hCVF for use according to claim 1, 2, or 3, wherein the hCVF is selected from the group consisting of HC3-1550, HC3-1348, HC3-1496, and HC3-1504.

5. hCVF for use according to claim 1, wherein the clotting factor is selected from the group consisting of a purified polypeptide, recombinant polypeptide, fusion polypeptide, peptide, aptamer, protein, antibody conjugate.

6. hCVF for use according to claim 1, wherein the clotting factor is Factor VIII.

7. hCVF for use according to claim 1, wherein the administration is selected from the group consisting of administration into an organ, into a cavity, into a tissue, intravenous, intraperitoneal, intraarterial, and subcutaneous administration.

8. Humanized cobra venom factor (hCVF) for use in reducing or preventing an immune response to a biologic therapeutic agent in a subject having a blood clotting_disorder associated with unwanted complement activation and to whom the biologic therapeutic agent is administered.

9. hCVF for use according to claim 8, wherein the hCVF is selected from the group consisting of HC3-1550, HC3-1348, HC3-1496, and HC3-1504.

10. hCVF for use according to claim 8 or 9, wherein administration of the hCVF is a second or subsequent administration to the subject and complement is depleted by said second or subsequent administration.

11. hCVF for use according to claim 8, whereby complement levels in the subject return to pre-hCVF administration levels within about 12 hours to 24 hours, within about 5 days to 7 days, or return in greater than 7 days.

12. hCVF according to claim 8, wherein the administration is selected from the group consisting of administration into an organ, into a cavity, into a tissue, intravenous, intraperitoneal, intraarterial, and subcutaneous administration.

## Patentansprüche

1. Humanisierter Kobragiftfaktor (hCVF, humanized Cobra Venom Factor) zur Verwendung bei der Verringerung oder Verhinderung einer Immunantwort auf einen Gerinnungsfaktor bei einem Patienten, dem der Gerinnungsfaktor verabreicht wird, wobei dem Patienten eine therapeutisch wirksame Menge des humanisierten Kobragiftfaktos (hCVF) und des Gerinnungsfaktors verabreicht wird, wodurch die Verabreichung von hCVF die Komplementspiegel im Blut des Patienten verringert.

2. hCVF zur Verwendung nach Anspruch 1, wobei die Verabreichung des Gerinnungsfaktors und des hCVF eine zweite oder eine nachfolgende Verabreichung an den Patienten ist und die Immunantwort durch die zweite oder nachfolgende Verabreichung verringert oder verhindert wird.

3. hCVF zur Verwendung nach Anspruch 1, wobei der hCVF vor der Verabreichung des Gerinnungsfaktors verabreicht wird.

4. hCVF zur Verwendung nach Anspruch 1, 2 oder 3, wobei der hCVF aus der Gruppe bestehend aus HC3-1550, HC3-1348, HC3-1496 und HC3-1504 ausgewählt ist.

5. hCVF zur Verwendung nach Anspruch 1, wobei der Gerinnungsfaktor aus der Gruppe bestehend aus einem gereinigten Polypeptid, rekombinantem Polypeptid, Fusionspolypeptid, Peptid, Aptamer, Protein, Antikörperkonjugat ausgewählt ist.

6. hCVF zur Verwendung nach Anspruch 1, wobei der Gerinnungsfaktor Faktor VIII ist.

7. hCVF zur Verwendung nach Anspruch 1, wobei die Verabreichung aus der Gruppe bestehend aus Verabreichung in ein Organ, in einen Hohlraum, in ein Gewebe, intravenöse, intraperitoneale, intraarterielle und subkutane Verabreichung ausgewählt ist.

8. Humanisierter Kobragiftfaktor (hCVF) zur Verwendung bei der Verringerung oder Verhinderung einer Immunantwort auf ein biologisches Therapeutikum bei einem Patienten mit einer Blutgerinnungsstörung, die mit einer unerwünschten Komplementaktivierung verbunden ist, und dem das biologische Therapeutikum verabreicht wird.

9. hCVF zur Verwendung nach Anspruch 8, wobei der hCVF aus der Gruppe bestehend aus HC3-1550, HC3-1348, HC3-1496 und HC3-1504 ausgewählt ist.

10. hCVF zur Verwendung nach Anspruch oder 8 oder 9, wobei die Verabreichung des Gerinnungsfaktors und des hCVF eine zweite oder eine nachfolgende Verabreichung an den Patienten ist und das Komplement durch die zweite oder nachfolgende Verabreichung erschöpft wird.

11. hCVF zur Verwendung nach Anspruch 8, wobei die Komplementwerte in dem Patienten innerhalb von etwa 12 Stunden bis 24 Stunden, innerhalb von etwa 5 Tagen bis 7 Tagen oder innerhalb von mehr als 7 Tagen zu den Werten vor der hCVF-Verabreichung zurückkehren.

12. hCVF nach Anspruch 8, wobei die Verabreichung aus der Gruppe bestehend aus Verabreichung in ein Organ, in einen Hohlraum, in ein Gewebe, intravenöse, intraperitoneale, intraarterielle und subkutane Verabreichung ausgewählt ist.

## Revendications

1. Facteur de venin de cobra humanisé (hCVF) destiné à être utilisé pour réduire ou prévenir une réponse immunitaire à un facteur de coagulation chez un sujet auquel le facteur de coagulation est administré, où une quantité thérapeutiquement efficace du facteur de venin de cobra humanisé (hCVF) et dudit facteur de coagulation est administrée au sujet, faisant en sorte que l'administration de hCVF entraîne une déplétion des taux de complément dans le sang du sujet.

2. hCVF destiné à être utilisé selon la revendication 1, où l'administration du facteur de coagulation et du hCVF constitue une deuxième administration ou une administration subséquente chez le sujet et la réponse immunitaire est réduite ou évitée par ladite deuxième administration ou administration subséquente.

3. hCVF destiné à être utilisé selon la revendication 1, ledit hCVF étant administré avant l'administration du facteur de coagulation.

4. hCVF destiné à être utilisé selon la revendication 1, 2 ou 3, où le hCVF est choisi dans le groupe constitué par HC3-1550, HC3-1348, HC3-1496 et HC3-1504.

5. hCVF destiné à être utilisé selon la revendication 1, où le facteur de coagulation est choisi dans le groupe constitué par un polypeptide purifié, un polypeptide recombinant, un polypeptide de fusion, un peptide, un aptamère, une protéine, un conjugué d'anticorps.

6. hCVF destiné à être utilisé selon la revendication 1, où le facteur de coagulation est le Facteur VIII.

7. hCVF destiné à être utilisé selon la revendication 1, où l'administration est choisie dans le groupe constitué par l'administration dans un organe, dans une cavité, dans un tissu, l'administration intraveineuse, intrapéritonéale, intra-artérielle et sous-cutanée.

8. Facteur de venin de cobra humanisé (hCVF) destiné à être utilisé pour réduire ou prévenir une réponse immunitaire à un agent thérapeutique biologique chez un sujet ayant un trouble de la coagulation sanguine associé à une activation inattendue du complément et auquel l'agent thérapeutique biologique est administré.

9. hCVF destiné à être utilisé selon la revendication 8, ledit hCVF étant choisi dans le groupe constitué par HC3-1550, HC3-1348, HC3-1496 et HC3-1504.

10. hCVF destiné à être utilisé selon la revendication 8 ou 9, où l'administration du hCVF constitue une deuxième administration ou une administration subséquente chez le sujet et ladite deuxième administration ou administration subséquente entraîne une déplétion du complément.

11. hCVF destiné à être utilisé selon la revendication 8, où les taux de complément chez le sujet sont revenus aux taux avant l'administration de hCVF dans un délai d'environ 12 heures à 24 heures, d'environ 5 jours à 7 jours, ou sont revenus dans un délai supérieur à 7 jours.

12. hCVF selon la revendication 8, où l'administration est choisie dans le groupe constitué par l'administration dans un organe, dans une cavité, dans un tissu, l'administration intraveineuse, intrapéritonéale, intra-artérielle et sous-cutanée.
